# EUROPEAN PATENT SPECIFICATION

(11) **EP 2 995 295 B1**
(45) Date of publication and mention of the grant of the patent: **16.10.2019**
(21) Application number: 14794248.6
(22) Date of filing: 01.05.2014
(51) Int. Cl.: A61K 8/898, A61K 8/894, A61Q 1/02, A61Q 1/10, A61Q 17/04, C09K 3/00

(54) **OIL-BASED THICKENING AGENT, OIL-BASED THICKENING COMPOSITION, AND COSMETIC PREPARATION**
ÖLBASIERTES VERDICKUNGSMITTEL, ÖLBASIERTE VERDICKUNGSZUSAMMENSETZUNG UND KOSMETISCHE ZUBEREITUNG
AGENT ÉPAISSISSANT À BASE D'HUILE, COMPOSITION ÉPAISSISSANTE À BASE D'HUILE, ET PRÉPARATION COSMÉTIQUE

(30) Priority: 09.05.2013 JP 2013099496
(43) Date of publication of application: 16.03.2016
(73) Proprietor: Shin-Etsu Chemical Co., Ltd., Tokyo 100-0004 (JP)
(72) Inventor: KIKUCHI Hiroko, Tokyo 100-0004 (JP)
(74) Representative: Mewburn Ellis LLP
(86) International application number: PCT/JP2014/062044
(87) International publication number: WO 2014/181747

(56) References cited:
- WO-A1-2012/133293
- JP-A- 2005 325 088
- JP-A- 2013 079 211
- US-A1- 2011 150 800
- Michael S Starch ET AL: "Beyond rheology modification: hydrophilically modified silicone elastomers provide new benefits", Journal of cosmetic science, 1 March 2003 (2003-03-01), pages 193-205, XP055312760, United States Retrieved from the Internet: URL:http://www.abcdef.com.ua/fotky27371/N_ 05p00193-p00205.pdf
- F Alan ET AL: "Scientific Literature Review Dimethicone Crosspolymers Ingredients as Used in Cosmetics The 2012 Cosmetic Ingredient Review Expert Panel members are: Chair", , 16 February 2012 (2012-02-16), XP055312905, Retrieved from the Internet: URL:http://www.cir-safety.org/sites/defaul t/files/Dimeth032012SLR_forposting.pdf

## Description

### TECHNICAL FIELD

The present invention relates an oil-based thickening agent, especially an oil-based thickening agent which has a large thickening effect on oil-based ingredients and is capable of forming oil-based compositions that are pleasant to use; an oil-based thickening composition which contains the oil-based thickening agent and a liquid oil component; and a cosmetic preparation formulated with the oil-based thickening agent or the oil-based thickening composition.

In this invention, "oil-based thickening composition" refers to a composition which contains a specific oil-based thickening agent and a liquid oil component, which composition overall is of increased viscosity (thickened) compared with the viscosities of the respective compositions obtained by adding and blending, into the liquid oil component, the silicone-modified polysaccharide compound alone or the silicone emulsifying agent alone which are contained in the oil-based thickening agent.

### BACKGROUND ART

Because they spread easily and have a clean and light feel, silicone oils are used in a variety of cosmetic preparations and quasi-drugs, including skin care cosmetics, makeup cosmetics and hair cosmetics. At the same time, cosmetic preparations that use oil-based thickening agents include, for example, liquid foundations, sunscreen gels, moisturizing creams, hair gels and antiperspirant creams. However, there are substantially no oil-based thickening agents capable of thickening silicone oils and other oil-based ingredients which are satisfactory in terms of all relevant qualities such as thickening effect, pleasantness of use and stability.

JP-A H05-311076 discloses that a polyether-modified silicone thickens silicone oil components in the presence of water.

However, when a polyether-modified silicone is used alone in the absence of water, the silicone oil thickening effect is very limited.

JP-A H08-208989 discloses that silicone-modified pullulan, which is a silicone-modified polysaccharide, thickens silicone oil components, but this too has an inadequate thickening effect.

JP-A-2005/325088 discloses skin care preparations containing a silicone-modified polysaccharide and inorganic powder. Embodiments also contain polyoxyethylene methylpolysiloxane copolymer and silicone oil.

JP-A-2013/079211 and WO2012/133293 disclose eyelash cosmetics containing dextrin fatty acid ester and siliconized polysaccharide, such as the pullulan derivative TSPL (tri(trimethylsiloxy)silyl-propylcarbamoyl pullulan). Examples also contain PEG-10 dimethicone and silicone oil.

When polyether-modified silicones or silicone-modified polysaccharide compounds are included in large amounts within oil-based compositions or cosmetic preparations so as to increase the thickening effects, they are tacky or otherwise affect the pleasantness of use. Moreover, although both elicit thickening effects, those effects are inadequate.

### SUMMARY OF THE INVENTION

### PROBLEMS TO BE SOLVED BY THE INVENTION

It is therefore an object of the present invention to provide an oil-based thickening agent which has a high thickening effect on silicone oils and other oil-based ingredients and is capable of forming oil-based compositions that are pleasant to use; an oil-based thickening composition which contains this oil-based thickening agent and a liquid oil component; and a cosmetic preparation formulated with the oil-based thickening agent or the oil-based thickening composition.

### MEANS FOR SOLVING THE PROBLEMS

The inventors have conducted extensive investigations, as a result of which they have discovered that, by using an oil-based thickening agent which includes both a silicone emulsifying agent and a silicone-modified polysaccharide of formula (1), there can be obtained an oil-based thickening composition which has a high thickening effect on oil-based ingredients and which also is very pleasant to use.

Accordingly, the invention provides the following oil-based thickening agent, oil-based thickening composition and cosmetic preparation.

According to claim 1, an oil-based thickening agent comprising
(a) a silicone-modified polysaccharide compound of the general formula (1): 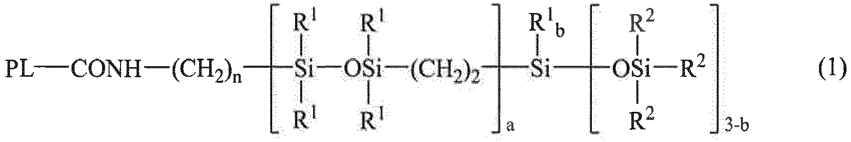 wherein PL is a glucose residue of pullulan; R¹ are identical or different and are unsubstituted or substituted monovalent hydrocarbon group of 1 to 10 carbon atoms; R² are identical or different and are unsubstituted or substituted monovalent hydrocarbon group of 1 to 10 carbon atoms or a siloxy group of the formula -OSi(R³)₃; R³ are identical or different and are unsubstituted or substituted monovalent hydrocarbon group of 1 to 8 carbon atoms; "n" is an integer from 1 to 10, "a" is 0 or 1, and "b" is 0, 1 or 2; and the average bonding number or degree of substitution of silicone compound per constituent sugar unit on the polysaccharide compound is from 0.5 to 2.5, and wherein the silicone-modified polysaccharide compound has an average molecular weight of from 50,000 to 10,000,000, and
(b) one or more silicone emulsifying agents selected from
   - dimethicone/PEG-10/15 crosspolymer,
   - PEG-15/lauryl dimethicone crosspolymer,
   - PEG-10/lauryl dimethicone crosspolymer,
   - PEG-15/lauryl polydimethylsiloxyethyl dimethicone crosspolymer,
   - polyglyceryl-3 polydimethylsiloxyethyl dimethicone,
   - lauryl polyglyceryl-3 polydimethylsiloxyethyl dimethicone,
   - bis-butyl dimethicone polyglyceryl-3,
   - dimethicone/polyglycerin-3 crosspolymer,
   - lauryl dimethicone/polyglycerin-3 crosspolymer, and
   - polyglyceryl-3/lauryl polydimethylsiloxyethyl dimethicone crosspolymer.

The silicone-modified polysaccharide compound may be a silicone-modified pullulan of the general formula (5) below: 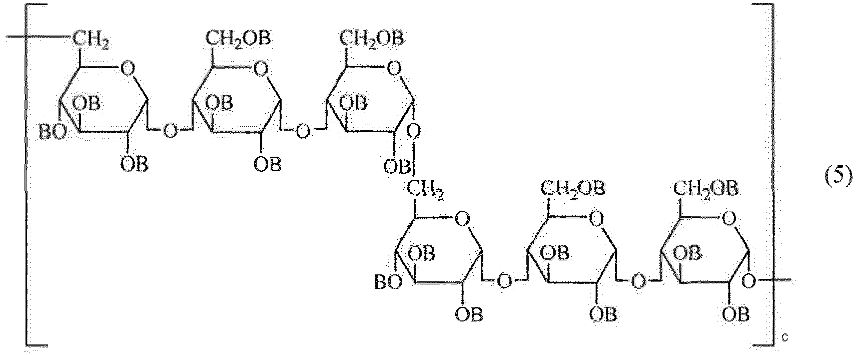 wherein B is a hydrogen atom or -CONH(CH₂)₃Si[OSi(CH₃)]₃, the degree of substitution is from 0.5 to 2.5, and "c" is a number from 100 to 20,000.

According to claim 5, an oil-based thickening composition characterized by comprising an oil-based thickening agent as defined above and an oil-based ingredient.

The oil-based ingredient may be a liquid oil component.

The oil-based liquid component may be selected from among low-viscosity silicone oils having a kinetic viscosity of not more than 50 mm²/s, light isoparaffin and light liquid isoparaffin.

According to claim 11, a cosmetic preparation formulated with the above oil-based thickening agent and/or the above oil-based thickening composition.

According to claim 12, the use of the above oil-based thickening agent and/or of the above oil-based thickening composition for formulating a cosmetic preparation.

### ADVANTAGEOUS EFFECTS OF THE INVENTION

According to this invention, by using an oil-based thickening agent which includes both a silicone emulsifying agent and a silicone-modified polysaccharide compound, there can be obtained an oil-based composition which exhibits a higher thickening effect on silicone oils and other oil-based ingredients than in cases where a silicone emulsifying agent or a silicone-modified polysaccharide compound is used alone. The resulting oil-based composition lacks tackiness and is pleasant to use, and thus lends itself well to use in cosmetic preparations.

In particular, by using one, two or more liquid oil components selected from among low-viscosity silicone oils having a kinetic viscosity of not more than 50 mm²/s, light isoparaffin and light liquid isoparaffin, an easy spreadability and a light, clean feel can be imparted to the oil-based thickening composition of the invention or the cosmetic preparation of the invention formulated with such an oil-based thickening agent or oil-based thickening composition.

### EMBODIMENTS FOR CARRYING OUT THE INVENTION

The oil-based thickening agent of the invention is composed of a silicone-modified polysaccharide compound of the general formula (1) below having an average molecular weight of from 50,000 to 10,000,000, and a silicone emulsifying agent. 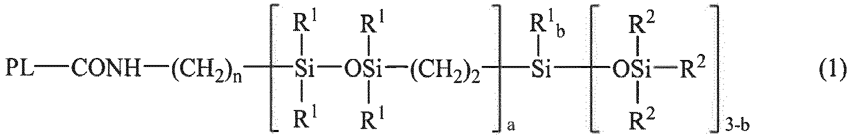 In formula (1), PL is a glucose residue of pullulan; R¹ is identical or different and an unsubstituted or substituted monovalent hydrocarbon group of 1 to 10 carbon atoms; R² is identical or different and an unsubstituted or substituted monovalent hydrocarbon group of 1 to 10 carbon atoms or a siloxy group of the formula -OSi(R³)₃; and R³ is identical or different and an unsubstituted or substituted monovalent hydrocarbon group of 1 to 8 carbon atoms. Also, "n" is an integer from 1 to 10, "a" is 0 or 1, and "b" is 0, 1 or 2. The average bonding number (degree of substitution) of silicone compound per constituent sugar unit on the polysaccharide compound is from 0.5 to 2.5.

### Silicone-Modified Polysaccharide Compound

The silicone-modified polysaccharide compound used in this invention is shown in the general formula (1) above.

In the general formula (1), PL is a glucose residue of pullulan. R¹ is identical or different and an unsubstituted or substituted monovalent hydrocarbon group of 1 to 10 carbon atoms, preferably 1 to 8 carbon atoms. R² is identical or different and an unsubstituted or substituted monovalent hydrocarbon group of 1 to 10 carbon atoms, preferably 1 to 8 carbon atoms, or a siloxy group of the formula -OSi(R³)₃, with R³ being identical or different and an unsubstituted or substituted monovalent hydrocarbon group of 1 to 8 carbon atoms, preferably 1 to 6 carbon atoms. Illustrative examples of these unsubstituted or substituted monovalent hydrocarbon groups R¹ to R³ include alkyl groups such as methyl, ethyl, propyl, isopropyl, butyl, isobutyl, tert-butyl, pentyl, hexyl, cyclohexyl, heptyl and octyl groups; alkenyl groups such as vinyl and allyl groups; aryl groups such as phenyl groups; and aralkyl groups such as benzyl groups; as well as any of these groups in which some or all of the hydrogen atoms have been substituted with, for example, halogen atoms such as fluorine, bromine or chlorine, or cyano groups. Examples include chloromethyl, chloropropyl, bromoethyl, trifluoropropyl and cyanoethyl groups. In terms of, for example, ease of synthesis and stability of the compound, it is desirable for these unsubstituted or substituted monovalent hydrocarbon groups represented by R¹ to R³ to be preferably unsubstituted or halogen-substituted monovalent hydrocarbon groups, especially unsubstituted monovalent hydrocarbon groups, and more preferably alkyl groups or aryl groups, especially methyl, ethyl or phenyl groups.

Also, "n" is an integer from 1 to 10, and preferably an integer from 1 to 6; "a" is 0 or 1, and preferably 0; and "b" is 0, 1 or 2, and preferably 0.

In the silicone-modified polysaccharide compound of the general formula (1) above, the average bonding number or degree of substitution of silicone compound per constituent sugar unit on the polysaccharide compound (pullulan) is from 0.5 to 2.5, and preferably from 1.0 to 2.0. When the average bonding number is too small, a sufficient oil solubility is not obtained; when it is too large, formation of the compound itself becomes difficult. The silicone compound of the general formula (2) below: 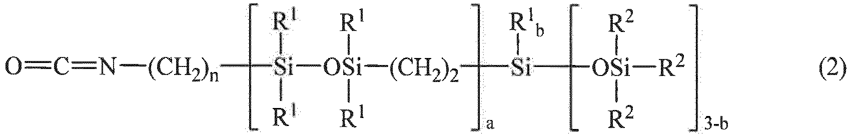 wherein R¹, R², "n", "a" and "b" are the same as above.

In this invention, the "degree of substitution of a silicone-modified polysaccharide" refers to the average bonding number of silicone compound per constituent sugar unit on the polysaccharide compound. For example, the degree of substitution of the silicone-modified polysaccharide compound of the general formula (1) indicates the average number of the substituents shown in the general formula (4) below that are attached to the basic unit of pullulan shown in formula (3) below: 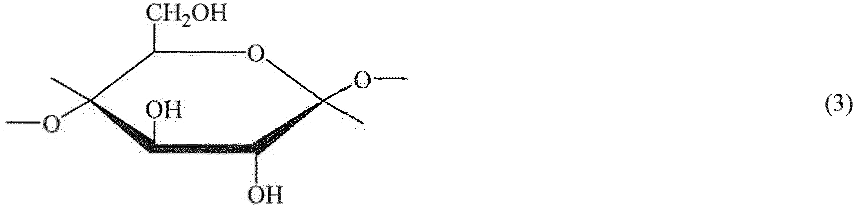 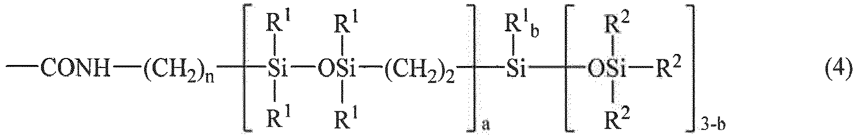 wherein R¹, R², "n", "a" and "b" are the same as above.

Also, the silicone-modified polysaccharide compound in this invention has an average molecular weight of from 50,000 to 10,000,000, and preferably from 80,000 to 5,000,000. The average molecular weight can typically be determined as, for example, the polystyrene-equivalent number average molecular weight or weight average molecular weight in gel permeation chromatography analysis using toluene, tetrahydrofuran (THF) as the developing solvent.

In the silicone-modified polysaccharide compound of the invention, it is especially preferable for n = 3, a = 0, b = 0 and R² to be methyl groups. Examples of such preferred silicone-modified polysaccharide compounds (i.e., cases in which n = 3, a = 0, b = 0, and R² represents methyl groups) include the silicone-modified pullulan shown in the general formula (5) below. 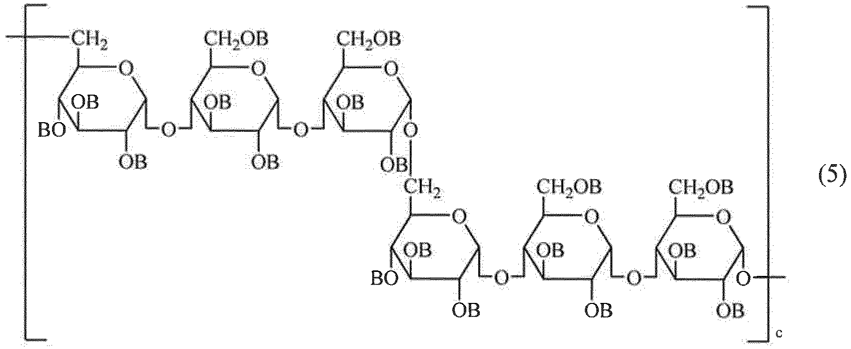 In the general formula (5), B is a hydrogen atom or a group of the formula -CONH(CH₂)₃Si[OSi(CH₃)₃]₃, the degree of substitution is from 0.5 to 2.5, and "c" is a number from 100 to 20,000.

The silicone-modified polysaccharide compound of formula (1) above that is used in this invention can be obtained by reacting an isocyanate-terminated silicone compound of the general formula (2) above with a polysaccharide compound (pullulan). A hitherto known method, such as that described in JP-A H08-134103, may be used to carry out such a reaction between a silicone compound and a polysaccharide compound.

The silicone-modified polysaccharide compound used in this invention can be obtained by a known method such as that mentioned above. For example, use may be made of the tri(trimethylsiloxy)silylpropylcarbamoyl pullulan (TSPL) defined in the Japanese Cosmetic Ingredient Labeling Name Dictionary (JCLD), commercial forms of which include TSPL-30-D5, which is TSPL dissolved in decamethylcyclopentasiloxane (available from Shin-Etsu Chemical Co., Ltd.), and TSPL-30-ID, which is TSPL dissolved in isododecane (available from Shin-Etsu Chemical Co., Ltd.).

### Silicone Emulsifying Agent

In the oil-based thickening agent of this invention, the thickening effect on oil-based ingredients is thought to appear as a result of the formation of a structure due to hydrogen bonding between hydrophilic groups on the silicone emulsifying agent and hydroxyl groups and urethane linkages (-OC(=O)NH-) on the silicone-modified polysaccharide compound. The silicone emulsifying agent used in this invention is capable of forming this structure. Use can be made of a silicone emulsifying agent that is known to the field of the invention.

The silicone emulsifying agents are exemplified by polyoxyalkylene-modified silicones, polyglycerol-modified silicones. In cases where an especially high thickening effect is desired, the use of one, two or more selected from among the polyoxyalkylene-modified silicones and polyglycerol-modified silicones is preferred.

The polyoxyalkylene-modified silicones that may be used are the following defined in the Japanese Cosmetic Ingredient Labeling Name Dictionary (JCLD): Dimethicone/PEG-10/15 Crosspolymer, PEG-15/Lauryl Dimethicone Crosspolymer, PEG-10/Lauryl Dimethicone Crosspolymer and PEG-15/Lauryl Polydimethylsiloxyethyl Dimethicone Crosspolymer. These may be used as mixtures with an optional oil-based ingredient.

Commercial products include the following, available from Shin-Etsu Chemical Co., Ltd.: KSG-210, KSG-240, KSG-310, KSG-320, KSG-330, KSG-340, KSG-320Z and KSG-350Z.

The polyglycerol-modified silicones that may be used are the following defined in the Japanese Cosmetic Ingredient Labeling Name Dictionary (JCLD): Polyglyceryl-3 Polydimethylsiloxyethyl Dimethicone, Lauryl Polyglyceryl-3 Polydimethylsiloxyethyl Dimethicone, Bis-Butyl Dimethicone Polyglyceryl-3, Dimethicone/Polyglycerin-3 Crosspolymer, Lauryl Dimethicone/Polyglycerin-3 Crosspolymer and Polyglyceryl-3/Lauryl Polydimethylsiloxyethyl Dimethicone Crosspolymer. These may be used as mixtures with an optional oil-based ingredient.

Commercial products include the following, available from Shin-Etsu Chemical Co., Ltd.: KF-6104, KF-6105, KF-6109, KSG-710, KSG-810, KSG-820, KSG-830, KSG-840, KSG-820Z and KSG-850Z.

### Oil-Based Thickening Agent

The oil-base thickening agent of the invention is made up of the above silicone emulsifying agent and silicone-modified polysaccharide compound. Its thickening effect on oil-based ingredients is conspicuously high compared with cases in which the silicone emulsifying agent or the silicone-modified polysaccharide compound is used alone.

In the oil-based thickening agent of the invention, a thickening effect emerges when the silicone emulsifying agent and the silicone-modified polysaccharide compound are combined in any ratio. The ratio, expressed as silicone emulsifying agent: silicone-modified polysaccharide compound, is not particularly limited and may be varied over a broad range of from 0.5:99.5 to 99.5:0.5. The ratio of silicone emulsifying agent to silicone-modified polysaccharide compound is preferably from 1:4 to 4:1, and more preferably from 2:3 to 3:2.

### Oil-Based Thickening Composition

The oil-based thickening composition of the invention contains the oil-based thickening agent obtained above and an oil-based ingredient. The inventive oil-based thickening agent that uses a silicone emulsifying agent and a silicone-modified polysaccharide compound is able to thicken oil-based compositions or various oil-based ingredients formulated in cosmetic preparations. The various oil-based ingredients are not particularly limited, provided they are capable of dissolving the silicone emulsifying agent and the silicone-modified polysaccharide compound. Examples include oil-based ingredients commonly used in cosmetic preparations and external preparations for the skin, such as silicone oils, hydrocarbon oils, higher fatty acids, higher alcohols, ester oils, natural fatty oils, waxes and the like.

A liquid oil component may be suitably selected from among such liquid-based ingredients, particularly in cases where it is desired that the oil-based thickening composition which can be formulated with the oil-based thickening agent of the invention, or a cosmetic preparation formulated with the oil-based thickening agent or the oil-based thickening composition, spreads easily and has a clean, light feel. It is more preferable to use one, two or more selected from among low-viscosity silicone oils having a kinetic viscosity at 25°C of not more than 50 mm²/s, and especially from 0.65 to 20 mm²/s, light isoparaffin and light liquid isoparaffin. The kinetic viscosity can be measured with a capillary kinematic viscometer.

Illustrative examples of such liquid oil components include the following defined in the Japanese Cosmetic Ingredient Labeling Name Dictionary (JCLD): Dimethicone, Cyclomethicone, Cyclopentasiloxane, Cyclohexasiloxane, Methyl Trimethicone, Diphenyl Dimethicone, Diphenylsiloxy Phenyl Trimethicone, Isododecane and Isohexadecane.

Commercial products include the KF-96 series, KF-995, TMF-1.5 and KF-56A, all available from Shin-Etsu Chemical Co., Ltd.; and Marukasol R, available from Maruzen Petrochemical Co., Ltd.

The oil-based thickening agent of the invention exhibits high thickening effects on these low-viscosity silicone oils, light isoparaffin and light liquid isoparaffin, enabling oil-based thickening compositions that are pleasant to use and have a good stability to be formed.

The oil-based thickening composition in this invention refers to a composition having a viscosity at least as large as (or larger than) when the silicone emulsifying agent and the silicone-modified polysaccharide compound are each dissolved alone in the liquid oil component. So long as the state of the composition has thickened, this includes compositions in the state of liquid substances ranging from low viscosity to high viscosity, and compositions in a gel state in which the liquid fluidity has been lost.

Preparation of the oil-based thickening composition of the invention is easy when the silicone emulsifying agent and the silicone-modified polysaccharide compound are separately dissolved in a low-viscosity silicone oil, light isoparaffin, light liquid paraffin or the like, and then mixed together. If necessary, heat may be applied during dissolution and mixture.

The amount of the oil-based thickening agent of the invention that is included in the oil-based composition may be selected as suitable for the intended purpose. Typically, from 0.1 to 80 wt % may be included, although the content is preferably from 3 to 50 wt %, and more preferably from 5 to 30 wt %. When the content is too low, sufficient effects are not exhibited. On the other hand, a content that is too high may make the composition tacky or otherwise less pleasant to use.

### Cosmetic Preparation

The inventive oil-based thickening agent composed of a silicone emulsifying agent and a silicone-modified polysaccharide compound is able, in small amounts, to thicken various oil-based ingredients, and can be advantageously used in cosmetic preparations without tackiness due to the thickening agent.

An oil-based thickening agent composed of a silicone emulsifying agent and a silicone-modified polysaccharide compound, or an oil-based composition obtained by adding various types of oil-based ingredients to an oil-based thickening agent, can be used in the cosmetic preparation of the invention.

The amount of the inventive oil-based thickening agent included in the cosmetic preparation may be set as appropriate for the intended purpose. Typically, based on the weight of the overall cosmetic preparation, from 0.1 wt % to 80 wt % may be included, with from 1 wt % to 50 wt % being preferred, and from 2 wt % to 30 wt % being especially preferred. When too little is included, a sufficient thickening effect may not be exhibited. On the other hand, when too much is included, this may impart undesirable effects such as tackiness to the cosmetic preparation.

The above-described oil-based composition may be used directly as the cosmetic preparation of the invention. However, in addition to the essential ingredients of the oil-based composition, where necessary, ingredients that are typically used in cosmetic preparations, such as moisturizers, ultraviolet absorbers, fragrances, antioxidants, preservatives, extender pigments, color pigments, alcohols and water, may be suitably included.

As to the form of the cosmetic preparation, the composition may be rendered into a nonaqueous system or into an oil-in-water or water-in-oil emulsified composition.

The cosmetic preparation is exemplified by skin care cosmetics such as lotions, emulsions, creams and serums; makeup cosmetics such as foundations, makeup bases, lipsticks, rouge, eye shadow, mascara and eyeliners; skin cleansers such as body soaps, face washes and makeup removers; hair cleansers such as shampoos; hair cosmetics such as rinses, hair treatments and hair growth preparations; and also hair dyes, sunscreen cosmetics and nail care cosmetics.

### EXAMPLES

Working Examples, Comparative Examples and example formulations are given below to more concretely illustrate the invention, although the invention is not limited by these Examples. Unless noted otherwise, the formulated amounts are indicated in % (wt %). The test methods used in the invention were as follows.

### (1) Viscosity

Samples having a viscosity of less than 1,000 mPa·s were measured with a Brookfield rotary viscometer (TV-10 viscometer (Toki Sangyo Co., Ltd.), rotor No. 2; speed, 60 rpm) .

Samples having a viscosity of from 1,000 to 100,000 mPa·s were measured with a Brookfield rotary viscometer (TV10 viscometer (Toki Sangyo Co., Ltd.), rotor No. 4; speed, 6 rpm).

Samples having a viscosity of more than 100,000 mPa·s were measured with a Brookfield rotary viscometer (RVDV-III Ultra viscometer (Brookfield Engineering), spindle T-F; speed, 6 rpm).

### (2) Appearance

The sample was visually examined at room temperature (25°C) and judged to be a solid when in a fluid state and a gel when in a non-fluid state.

### WORKING EXAMPLES AND COMPARATIVE EXAMPLES

### Experiment 1. Synergistic Effects from Concomitant Use

In oil-based compositions containing an oil-based thickening agent that uses a silicone emulsifying agent alone, a silicone-modified polysaccharide compound alone, or both, and containing also, as the liquid oil component, a silicone oil (decamethylcyclopentasiloxane), the concentration of oil-based thickening agent in the compositions was varied and the viscosities and appearances of the compositions were compared.

The materials used were as follows.

### Silicone Emulsifying Agent (1):

Polyglycerin-3 Polydimethylsiloxyethyl Dimethicone (KF-6104, from Shin-Etsu Chemical Co., Ltd.)

### Silicone-Modified Polysaccharide Compound (1):

Tri(trimethylsiloxy)silylpropylcarbamoyl pullulan (TSPL, from Shin-Etsu Chemical Co., Ltd.; degree of substitution in general formula (2), approx. 2.0; molecular weight, approx. 690,000).

The results are shown in Table 1.

**Table 1**

| Composition (%) | Comparative Example 1 | Example 1 | Comparative Example 2 | Comparative Example 3 | Example 2 | Comparative Example 4 |
|---|---|---|---|---|---|---|
| Silicone Emulsifying Agent (1) | 15 | 6 | 0 | 30 | 12 | 0 |
| Silicone-Modified Polysaccharide Compound (1) | 0 | 9 | 15 | 0 | 18 | 30 |
| Decamethylcyclopentasiloxane | 85 | 85 | 85 | 70 | 70 | 70 |
| Oil-based thickening agent concentration (%) in composition | 15 | | | 30 | | |

| Evaluation Results | | | | | | |
|---|---|---|---|---|---|---|
| Viscosity (mPa·s) | 14.5 | 10,300 | 203.4 | 48.2 | 262,000 | 11,000 |
| Appearance | liquid | liquid | liquid | liquid | gel | liquid |

As shown in Table 1, at an oil-based thickening agent content in the composition of 15%, with the use of Silicone Emulsifying Agent (1) alone (Comparative Example 1) or Silicone-Modified Polysaccharide Compound (1) alone (Comparative Example 2), the viscosity of the oil-based composition was very low, resulting in a liquid state having fluidity. By contrast, with an oil-based thickening agent according to the invention that uses two ingredients (Example 1), even though the oil-based thickening agent content in the composition was 15%, which is low, the viscosity improved relative to compositions in which only one of the oil-based thickening agent ingredients was used (Comparative Examples 1 and 2), thus demonstrating a synergistic thickening effect.

At an oil-based thickening agent content in the composition of 30%, with the use of Silicone Emulsifying Agent (1) alone (Comparative Example 3), as in the case where the content was 15%, the viscosity was very low. With the use of a Silicone-Modified Polysaccharide Compound (1) alone (Comparative Example 4), despite the increase in the oil-based thickening agent content from 15% to 30%, although some degree of thickening effect was apparent, the composition remained in the state of a liquid having fluidity. By contrast, with an oil-based thickening agent according to the invention that uses two ingredients (Example 2), a very hard gel lacking fluidity formed, thus demonstrating a marked thickening effect.

Therefore, by using Silicone Emulsifying Agent (1) and Silicone-Modified Polysaccharide Compound (1) together as the oil-based thickening agent, the viscosity of the oil-based composition markedly improved compared with cases in which either was used alone.

### Experiment 2. Influence of Mixing Ratio

Aside from changing the mixing ratio of Silicone Emulsifying Agent (1) and Silicone-Modified Polysaccharide Compound (1), this investigation was carried out in the same way as in Experiment 1.

**Table 2**

| Composition (%) | Comparative Example 1 | Example 3 | Example 4 | Example 1 | Example 5 | Comparative Example 2 |
|---|---|---|---|---|---|---|
| Silicone Emulsifying Agent (1) | 15 | 12 | 9 | 6 | 3 | 0 |
| Silicone-Modified Polysaccharide Compound (1) | 0 | 3 | 6 | 9 | 12 | 15 |
| Decamethylcyclopentasiloxane | 85 | 85 | 85 | 85 | 85 | 85 |
| Oil-based thickening agent concentration (%) in composition | 15 | | | | | |

| Evaluation Results | | | | | | |
|---|---|---|---|---|---|---|
| Viscosity (mPa·s) | 14.5 | 1,900 | 9,400 | 10,300 | 3,000 | 203.4 |
| Appearance | liquid | liquid | liquid | liquid | liquid | liquid |

As shown in Table 2, oil-based thickening agents according to the invention which used two ingredients (Examples 1 and 3 to 5) had a synergistically improved viscosity over a broad range in mixing ratio.

### Experiment 3. Type of Silicone Emulsifying Agent

This investigation was carried out in the same way as Experiment 1, but using silicone emulsifying agents of different structures.

The new materials used were as follows.

### Silicone Emulsifying Agent (2):

PEG-10 Dimethicone
(KF-6017, from Shin-Etsu Chemical Co., Ltd.)

### Silicone Emulsifying Agent (3):

A mixture of about 24% of Dimethicone/PEG-10/15 Crosspolymer and about 76% of Methyl Polysiloxane (6 mm²/s) (KSG-210, from Shin-Etsu Chemical Co., Ltd.)

The results are shown in Table 3.

**Table 3**

| Composition (%) | Comparative Example 5 | Example 6 [Ref] | Comparative Example 6 | Example 7 | Comparative Example 7 | Example 8 [Ref] |
|---|---|---|---|---|---|---|
| Silicone Emulsifying Agent (2) | 15 | 6 | - | - | 30 | 12 |
| Silicone Emulsifying Agent (3) | - | - | 62.5¹ⁱ | 25²⁾ | - | - |
| Silicone-Modified Polysaccharide Compound (1) | 0 | 9 | 0 | 9 | 0 | 18 |
| Decamethylcyclopentasiloxane | 85 | 85 | 37.5 | 66 | 70 | 70 |
| Oil-based thickening agent concentration (%) in composition | 15 | | | | 30 | |

| Evaluation Results | | | | | | |
|---|---|---|---|---|---|---|
| Viscosity (mPa·s) | 13.3 | 3,400 | 2,800 | 104,000 | 48.6 | 50,300 |
| Appearance | liquid | liquid | liquid | gel | liquid | liquid |

| | | | | | | |
|---|---|---|---|---|---|---|
| 1) Concentration of Dimethicone/PEG-10/15 Crosspolymer in Silicone Emulsifying Agent (3) (KSG-210) was 15% 2) Concentration of Dimethicone/PEG-10/15 Crosspolymer in Silicone Emulsifying Agent (3) (KSG-210) was 6% 3) Examples 6 and 8 are reference Examples outside the claims | | | | | | |

As is apparent from Table 3, with regard to Silicone Emulsifying Agent (2), even when 15% (Comparative Example 5) or 30% (Comparative Example 7) of Silicone Emulsifying Agent (2) alone was included in the composition, the viscosity of the composition was very low. However, oil-based thickening agents according to the invention (Examples 6 and 8) that used Silicone Emulsifying Agent (2) and Silicone-Modified Polysaccharide Compound (1) together exhibited a high thickening effect. Moreover, as in Example 2, when 30% of an oil-based thickening agent according to the invention that is made up of the two ingredients was used (Example 8), an even higher thickening effect was exhibited.

Also, with regard to Silicone Emulsifying Agent (3), when Silicone Emulsifying Agent (3) was used alone (Comparative Example 6), this itself had an oil-based ingredient thickening effect. However, an oil-based thickening agent according to this invention (Example 7) that used Silicone Emulsifying Agent (3) and Silicone-Modified Polysaccharide Compound (1) together produced a hard gel, exhibiting a remarkable increase in viscosity.

Therefore, the effects of the invention are manifested between a plurality of types of silicone emulsifying agents and silicone-modified polysaccharide compounds.

### Experiment 4. Type of Liquid Oil Component

Other than using, as liquid oil components other than decamethylcyclopentasiloxane: Isododecane, Diphenylsiloxy Phenyl Trimethicone (KF-56A, from Shin-Etsu Chemical Co., Ltd.; viscosity, 15 mm²/s) and Methyl Polysiloxane (KF-96A-6CS, from Shin-Etsu Chemical Co., Ltd.; viscosity, 6 mm²/s), this investigation was carried out in the same way as in Experiment 1.

The results are shown in Table 4.

**Table 4**

| Composition (%) | Example 1 | Example 9 | Example 10 | Example 11 | Example 2 | Example 12 | Example 13 | Example 14 |
|---|---|---|---|---|---|---|---|---|
| Silicone Emulsifying Agent (1) | 6 | 6 | 6 | 6 | 12 | 12 | 12 | 12 |
| Silicone-Modified Polysaccharide Compound (1) | 9 | 9 | 9 | 9 | 18 | 18 | 18 | 18 |
| Decamethylcyclopentasiloxane | 85 | - | 21 | 21 | 70 | - | 42 | 42 |
| Isododecane | - | 85 | - | - | - | 70 | - | - |
| KF-56A | - | - | 64 | - | - | - | 28 | - |
| KF-96A-6CS | - | - | - | 64 | - | - | - | 28 |
| Oil-based thickening agent concentration (%) in composition | 15 | | | | 30 | | | |

| Evaluation results | | | | | | | | |
|---|---|---|---|---|---|---|---|---|
| Viscosity (mPa·s) | 10,300 | 1,900 | 5,400 | 5,600 | 262,000 | 29,200 | 142,300 | 145,700 |
| Appearance | liquid | liquid | liquid | liquid | gel | liquid | gel | gel |

As shown in Table 4, even when Isododecane, KF-56A, or KF-96A-6CS was used instead of decamethylcyclopentasiloxane as the liquid oil component, oil-based thickening agents according to the invention that used Silicone Emulsifying Agent (1) and Silicone-Modified Polysaccharide Compound (1) together (Examples 9 to 14) all exhibited sufficient thickening effects.

Examples in which cosmetic preparations were produced using an oil-based thickening agent composed of a silicone emulsifying agent and a silicone-modified polysaccharide compound are shown below.

**Table 5**

| Formulation Example 1: Emulsified liquid foundations | | | | | |
|---|---|---|---|---|---|
| | | 1-1 | 1-2 | 1-3 | 1-4 |
| (1) | Crosslinked polyether-modified silicone^{*1} | - | 3.5 | 3.5 | 3.5 |
| (2) | Crosslinked dimethylpolysiloxane^{*2} | 5.0 | 5.0 | 5.0 | 5.0 |
| (3) | Polyglycerol-modified silicone^{*3} | 4.0 | 2.0 | - | - |
| (4) | Branched polyether-modified silicone^{*4} | - | - | 2.0 | 10.0 |
| (5) | Dimethyldistearylammonium hectorite | 1.2 | 1.2 | 1.2 | 1.2 |
| (6) | Triethylhexanoin | 5.0 | 5.0 | 5.0 | 5.0 |
| (7) | Methyl polysiloxane (6 mm²/s) | 8.0 | 6.5 | 6.5 | 6.5 |
| (8) | Decamethylcyclopentasiloxane | 21.7 | 21.7 | 21.7 | 14.7 |
| (9) | 1,3-Butylene glycol | 5.0 | 5.0 | 5.0 | 5.0 |
| (10) | Sodium citrate | 0.2 | 0.2 | 0.2 | 0.2 |
| (11) | Sodium chloride | 0.5 | 0.5 | 0.5 | 0.5 |
| (12) | Purified water | 38.0 | 38.0 | 38.0 | 38.0 |
| (13) | Silicone-modified acrylic polymer^{*5} | 0.45 | 0.45 | 0.45 | 0.45 |
| (14) | Silicone-treated titanium oxide^{*6} | 8.50 | 8.50 | 8.50 | 8.50 |
| (15) | Silicone-treated red iron oxide^{*6} | 0.41 | 0.41 | 0.41 | 0.41 |
| (16) | Silicone-treated yellow iron oxide^{*6} | 0.97 | 0.97 | 0.97 | 0.97 |
| (17) | Silicone-treated black iron oxide^{*6} | 0.12 | 0.12 | 0.12 | 0.12 |
| (18) | Silicone-modified pullulan, 30% solution^{*7} | 1.0 | 1.0 | 1.0 | - |

| Evaluation results | | | | | |
|---|---|---|---|---|---|
| Viscosity (mPa·s) | | 6,200 | 12,600 | 29,000 | 20,000 |
| Feel | | Exc | Exc | Exc | NG |
| Stability | | good | Exc | Exc | good |

| | | | | | |
|---|---|---|---|---|---|
| *1: KSG-210, from Shin-Etsu Co., Ltd. *2: KSG-15, from Shin-Etsu Co., Ltd. *3: KF-6104, from Shin-Etsu Co., Ltd. *4: KF-6028P, from Shin-Etsu Co., Ltd. *5: KP-578, from Shin-Etsu Co., Ltd. *6: Treated with KF-9909, from Shin-Etsu Co., Ltd. *7: TSPL-30-D5, from Shin-Etsu Co., Ltd. | | | | | |

### Method of Evaluating Feel

Twenty expert panelists rated the feel in terms of the following five qualities.
1: Spreadability when applied
2: Tackiness
3: Evenness of finish
4: Sheerness of finish
5: Long-lasting (durability)

### Evaluation Criteria

Ratings were carried out based on the following criteria.

| | |
|---|---|
| Exc: | The foundation was satisfactory in terms of all five qualities. |
| Good: | Only one of the five qualities was less than satisfactory. |
| NG: | Two or more of the five qualities were less than satisfactory. |

### Method of Evaluating Stability

### High-temperature stability:

Evaluated after holding the foundation for 1 month in a 50°C thermostatic chamber.

### Normal-temperature stability:

Evaluated after holding the foundation for 1 month in a 50°C thermostatic chamber.

### Low-temperature stability:

Evaluated after holding the foundation for 1 month in a 50°C thermostatic chamber.

### Evaluation Criteria

Ratings were carried out based on the following criteria.

| | |
|---|---|
| Exc: | The stability was excellent at all three temperature levels. |
| Good: | The stability was good at all three temperature levels (even when the stability was excellent at up to two of the levels). |
| NG: | The stability was poor (discoloration, change in odor, separation) at one or more of the three temperature levels. |

Ingredients (1) to (8) were stirred and mixed to uniformity. In a separate step, ingredients (9) to (11) were uniformly dissolved in ingredient (12), following which the resulting solution was gently added to the mixture and emulsification was carried out. Ingredients (13) to (18), after being treated with rollers in a separate operation, were then added to and mixed with the emulsion. The resulting preparation was filled into a given container, giving an emulsified liquid foundation.

Formulations 1-1 to 1-4 were all prepared by the same method.

In Samples 1-1, 1-2 and 1-3 according to the invention in which a silicone emulsifying agent and a silicone-modified polysaccharide compound were used together, emulsified liquid foundations were obtained which, owing to the effects of the oil-based thickening agent, had a good stability, resisted running and smearing make-up due to human sebums (exhibited a high oil resistance), and were pleasant to use.

On the other hand, in Sample 1-4 formulated with only a silicone emulsifying agent, although the viscosity rose due to the increase in quantity of ingredient (4) and the stability was good, undesirable effects such as tackiness arose on account of the large amount of ingredient (4) added, and so this sample was judged to be unpleasant to use.

**Table 6**

| Formulation Example 2: Sunscreen Emulsion | | |
|---|---|---|
| Ingredients | | |
| (1) | Crosslinked polyether-modified silicone^{*1} | 3.0 |
| (2) | Crosslinked dimethylpolysiloxane^{*2} | 2.0 |
| (3) | Branched polyether-modified silicone^{*3} | 1.0 |
| (4) | Silicone-modified pullulan, 30% solution^{*4} | 3.0 |
| (5) | Decamethylcyclopentasiloxane | 5.0 |
| (6) | Dimethylpolysiloxane (6 mm²/s) | 2.0 |
| (7) | Isotridecyl isononanoate | 4.0 |
| (8) | Titanium oxide dispersion^{*5} | 25.0 |
| (9) | Zinc oxide dispersion^{*6} | 35.0 |
| (10) | 1,3-Butylene glycol | 2.0 |
| (11) | Sodium citrate | 0.2 |
| (12) | Sodium chloride | 1.0 |
| (13) | Purified water | 16.8 |

| | | |
|---|---|---|
| *1: KSG-210, from Shin-Etsu Co., Ltd. *2: KSG-15, from Shin-Etsu Co., Ltd. *3: KF-6028P, from Shin-Etsu Co., Ltd. *4: TSPL-30-D5, from Shin-Etsu Co., Ltd. *5: SPD-T5, from Shin-Etsu Co., Ltd. *6: SPD-Z5, from Shin-Etsu Co., Ltd. | | |

### Production of Cosmetic Preparation

| | |
|---|---|
| A: | Ingredients (1) to (7) were uniformly mixed together. |
| B: | Ingredients (10) to (13) were mixed together. |
| C: | B was added to A and emulsified, after which ingredients (8) and (9) were added, thereby giving a sunscreen emulsion. |

The sunscreen emulsion thus obtained spread easily, was not tacky or oily, and had a good resistance to water and perspiration.

**Table 7**

| Formulation Example 3: Sunscreen Lotion (shaking type) | | |
|---|---|---|
| *[Reference Example outside the claims]* | | |
| Ingredients | | |
| (1) | Branched polyether-modified silicone^{*1} | 2.0 |
| (2) | Silicone-modified pullulan, 30% solution^{*2} | 3.0 |
| (3) | Dimethylpolysiloxane (6 mm²/s) | 7.0 |
| (4) | Decamethylcyclopentasiloxane | 5.8 |
| (5) | Ethylhexyl methoxycinnamate | 7.5 |
| (6) | Silicone hybrid powder^{*3} | 0.5 |
| (7) | Dimethyldistearylammonium hectorite | 0.2 |
| (8) | Zinc oxide dispersion^{*4} | 45.0 |
| (9) | 1,3-Butylene glycol | 3.0 |
| (10) | Alcohol | 5.0 |
| (11) | Sodium citrate | 0.2 |
| (12) | Sodium chloride | 0.5 |
| (13) | Purified water | 20.3 |

| | | |
|---|---|---|
| *1: KF-6028P, from Shin-Etsu Co., Ltd. *2: TSPL-30-D5, from Shin-Etsu Co., Ltd. *3: KSP-105, from Shin-Etsu Co., Ltd. *4: SPD-Z6, from Shin-Etsu Co., Ltd. | | |

### Production of Cosmetic Preparation

| | |
|---|---|
| A: | Ingredients (1) to (7) were uniformly mixed together. |
| B: | Ingredients (9) to (13) were mixed together. |
| C: | B was added to A and emulsion was carried out, following which ingredient (8) was added, thereby giving a shaking type sunscreen lotion. |

The sunscreen lotion thus obtained spread easily, was not tacky or oily, and was very long-lasting when applied.

**Table 8**

| Formulation Example 4: Nonaqueous Mousse-type Foundation | | |
|---|---|---|
| Ingredients | | |
| (1) | Crosslinked polyether-modified silicone^{*1} | 18.00 |
| (2) | Dimethylpolysiloxane (6 mm²/s) | 12.00 |
| (3) | Neopentyl glycol dioctanoate | 5.00 |
| (4) | Silyl-treated silicic anhydride^{*2} | 0.75 |
| (5) | Silicone-treated red iron oxide^{*3} | 0.20 |
| (6) | Silicone-treated yellow iron oxide^{*3} | 1.00 |
| (7) | Silicone-treated black iron oxide^{*3} | 0.02 |
| (8) | Silicone-treated titanium oxide^{*3} | 5.00 |
| (9) | Silicone-treated talc^{*3} | 11.55 |
| (10) | Silicone-modified pullulan, 30% solution^{*4} | 3.00 |
| (11) | Trimethylsiloxysilicic acid, 50% solution^{*5} | 2.00 |
| (12) | Decamethylcyclopentasiloxane | 25.28 |
| (13) | Silicone hybrid powder^{*6} | 6.00 |
| (14) | Spherical polymethylsilsesquioxane^{*7} | 3.00 |
| (15) | Spherical poly(alkyl methacrylate) | 7.00 |
| (16) | Antioxidant | 0.20 |

| | | |
|---|---|---|
| *1: KSG-240, from Shin-Etsu Co., Ltd. *2: Aerosil R-972, a surface-hydrophobized fumed silica from Nippon Aerosil Co., Ltd. *3: Treated with KF-9909, from Shin-Etsu Co., Ltd. *4: TSPL-30-D5, from Shin-Etsu Co., Ltd. *5: KF-7312J, from Shin-Etsu Co., Ltd. *6: KSP-411, from Shin-Etsu Co., Ltd. *7: KMP-590, from Shin-Etsu Co., Ltd. | | |

### Production of Cosmetic Preparation

Ingredients (1) to (8) were uniformly mixed together by treatment with rollers. Ingredients (9) to (16) were then added and mixing was carried out to uniformity, thereby giving a nonaqueous mousse-type foundation.

The foundation thus obtained had a firmly hardened appearance in the form of a mousse, spread easily and was very pleasant to use, without tackiness or oiliness. Moreover, it was confirmed to be very long-lasting when applied.

**Table 9**

| Formulation Examples 5, 6 and 7: Mascara | | | | |
|---|---|---|---|---|
| *[Reference Examples, outside the claims]* | | | | |
| Ingredients | | | | |
| | | Formulation Example 5 | Formulation Example 6 | Formulation Example 7 |
| (1) | Silicone-modified pullulan, 30% solution^{*1} | 10.0 | 5.0 | 5.0 |
| (2) | Trimethylsiloxysilicic acid solution^{*2} | - | 15.0 | - |
| (3) | Acrylic silicone resin solution^{*3} | - | - | 15.0 |
| (4) | Dextrin palmitate/ethylhexanoate^{*4} | 3.0 | 3.0 | 3.0 |
| (5) | Ceresin | 2.5 | 2.5 | 2.5 |
| (6) | Behenyl-modified acrylic silicone resin^{*5} | 2.0 | 2.0 | 2.0 |
| (7) | Beeswax | 4.5 | 4.5 | 4.5 |
| (8) | Hydrogenated lecithin | 0.4 | 0.4 | 0.4 |
| (9) | Triethylhexanoin | 2.5 | 2.5 | 2.5 |
| (10) | Branched polyether-modified silicone^{*6} | 1.0 | 1.0 | 1.0 |
| (11) | Dimethyldistearylammonium hectorite | 4.0 | 4.0 | 4.0 |
| (12) | Propylene carbonate | 1.3 | 1.3 | 1.3 |
| (13) | Isododecane | 42.6 | 32.6 | 32.6 |
| (14) | Silicone-treated pigment^{*7} | 5.0 | 5.0 | 5.0 |
| (15) | Silicone-treated talc^{*7} | 4.5 | 4.5 | 4.5 |
| (16) | Silylated silicic anhydride^{*8} | 2.7 | 2.7 | 2.7 |
| (17) | 1,3-Butylene glycol | 1.0 | 1.0 | 1.0 |
| (18) | Preservative | as suitable | as suitable | as suitable |
| (19) | Purified water | balance | balance | balance |

| | | | | |
|---|---|---|---|---|
| *1: TSPL-30-ID, from Shin-Etsu Co., Ltd. *2: X-21-5595, from Shin-Etsu Co., Ltd. *3: KP-550, from Shin-Etsu Co., Ltd. *4: Rheopearl TT, from Chiba Flour Milling Co., Ltd. *5: KP-562P, from Shin-Etsu Co., Ltd. *6: KF-6028, from Shin-Etsu Co., Ltd. *7: Treated with KF-9909, from Shin-Etsu Co., Ltd. *8: Aerosil R-972, a surface-hydrophobized fumed silica from Nippon Aerosil Co., Ltd. | | | | |

### Production of Cosmetic Preparation

| | |
|---|---|
| A: | Ingredients (10) to (13) were uniformly mixed together. |
| B: | Ingredients (14) to (16) were added to A, followed by uniform mixture by treatment with rollers. |
| C: | Ingredients (1) to (9) were added to B, followed by warming and uniform mixture. |
| D: | Ingredients (17) to (19), which were uniformly mixed in a separate operation, were warmed then added to C, followed by stirring to effect mixture, thereby giving a mascara. |

Each of the mascaras thus obtained spread easily, readily adhered to the eyelashes, and was pleasant to use with no tackiness. In addition, secondary transfer did not readily occur (demonstrating a high rubbing resistance), and the mascara was confirmed to be very long-lasting.

Similar effects were confirmed to be achievable even when used together with silicone film formers commonly used in mascaras.

## Claims

1. An oil-based thickening agent comprising
(a) a silicone-modified polysaccharide compound of the general formula (1): 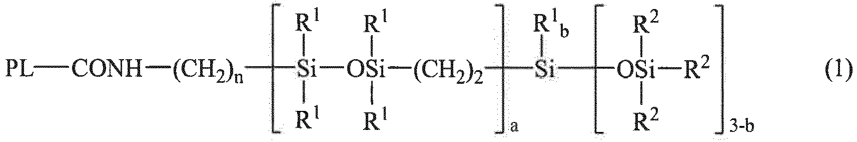 wherein PL is a glucose residue of pullulan; R¹ are identical or different and are unsubstituted or substituted monovalent hydrocarbon group of 1 to 10 carbon atoms; R² are identical or different and are unsubstituted or substituted monovalent hydrocarbon group of 1 to 10 carbon atoms or a siloxy group of the formula -OSi(R³)₃; R³ are identical or different and are unsubstituted or substituted monovalent hydrocarbon group of 1 to 8 carbon atoms; "n" is an integer from 1 to 10, "a" is 0 or 1, and "b" is 0, 1 or 2; and the average bonding number or degree of substitution of silicone compound per constituent sugar unit on the polysaccharide compound is from 0.5 to 2.5, and wherein the silicone-modified polysaccharide compound has an average molecular weight of from 50,000 to 10,000,000, and
(b) one or more silicone emulsifying agents selected from
- dimethicone/PEG-10/15 crosspolymer,
- PEG-15/lauryl dimethicone crosspolymer,
- PEG-10/lauryl dimethicone crosspolymer,
- PEG-15/lauryl polydimethylsiloxyethyl dimethicone crosspolymer,
- polyglyceryl-3 polydimethylsiloxyethyl dimethicone,
- lauryl polyglyceryl-3 polydimethylsiloxyethyl dimethicone,
- bis-butyl dimethicone polyglyceryl-3,
- dimethicone/polyglycerin-3 crosspolymer,
- lauryl dimethicone/polyglycerin-3 crosspolymer, and
- polyglyceryl-3/lauryl polydimethylsiloxyethyl dimethicone crosspolymer.

2. An oil-based thickening agent according to claim 1, wherein the silicone-modified polysaccharide compound is a silicone-modified pullulan of the general formula (5) below: 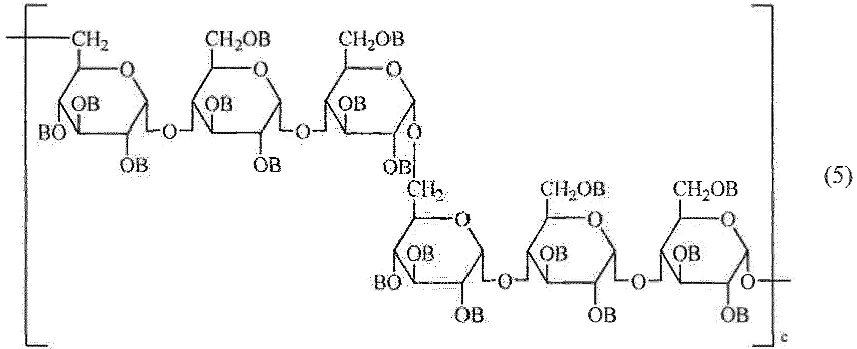 wherein B is a hydrogen atom or -CONH (CH₂) ₃Si[OSi(CH₃) ₃]₃, the degree of substitution is from 0.5 to 2.5, and "c" is a number from 100 to 20,000.

3. An oil-based thickening agent according to claim 1 or 2 wherein the ratio of silicone emulsifying agent to silicone-modified polysaccharide compound is from 0.5:99.5 to 99.5:0.5.

4. An oil-based thickening agent according to claim 3 wherein the ratio of silicone emulsifying agent to silicone-modified polysaccharide compound is from 1:4 to 4:1.

5. An oil-based thickening composition comprising an oil-based thickening agent of any one of claims 1 to 4 and an oil-based ingredient.

6. An oil-based thickening composition according to claim 5 wherein the oil-based ingredient is a liquid oil component.

7. An oil-based thickening composition according to claim 5 wherein the oil-based ingredient is a silicone oil.

8. An oil-based thickening composition according to claim 6 wherein the oil-based liquid component is selected from among low-viscosity silicone oils having a kinetic viscosity of not more than 50 mm²/s, light isoparaffin and light liquid isoparaffin.

9. An oil-based thickening composition according to any one of claims 5 to 8, consisting of said silicone-modified polysaccharide compound, silicone emulsifying agent and oil-based ingredient.

10. An oil-based thickening composition according to any one of claims 5 to 9 comprising from 5 to 30 wt% of the oil-based thickening agent consisting of said silicone-modified polysaccharide compound and silicone emulsifying agent.

11. A cosmetic preparation formulated with an oil-based thickening agent according to any one of claims 1 to 4 and/or an oil-based thickening composition according to any one of claims 5 to 10.

12. The use of an oil-based thickening agent according to any one of claims 1 to 4, and/or of an oil-based thickening composition according to any one of claims 5 to 10, for formulating a cosmetic preparation.

## Patentansprüche

1. Ölbasiertes Verdickungsmittel, das Folgendes umfasst:
(a) eine Silicon-modifizierte Polysaccharidverbindung der allgemeinen Formel (1): 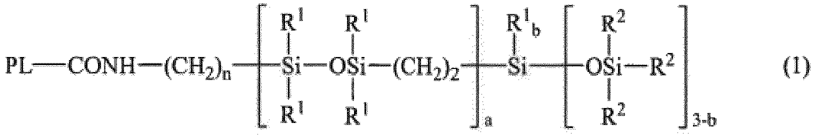 worin PL ein Glukoserest von Pullulan ist; die R¹ gleich oder verschieden sind und für eine unsubstituierte oder substituierte einwertige Kohlenwasserstoffgruppe mit 1 bis 10 Kohlenstoffatomen stehen; die R² gleich oder verschieden sind und für eine unsubstituierte oder substituierte einwertige Kohlenwasserstoffgruppe mit 1 bis 10 Kohlenstoffatomen oder eine Siloxygruppe der Formel -OSi(R³)₃ stehen; die R³ gleich oder verschieden sind und für eine unsubstituierte oder substituierte einwertige Kohlenwasserstoffgruppe mit 1 bis 8 Kohlenstoffatomen stehen; "n" eine ganze Zahl von 1 bis 10 ist, "a" = 0 oder 1 ist und "b" = 0, 1 oder 2 ist und die mittlere Bindungszahl oder der mittlere Substitutionsgrad der Siliconverbindung pro Grundzuckereinheit an der Polysaccharidverbindung 0,5 bis 2,5 beträgt und wobei die Silicon-modifizierte Polysaccharidverbindung ein mittleres Molekulargewicht von 50.000 bis 10.000.000 aufweist und
(b) einen oder mehrere Siliconemulgatoren ausgewählt aus:
- Dimethicon/PEG-10/15-Kreuzpolymer,
- PEG15/Lauryldimethicon-Kreuzpolymer,
- PEG-10/Lauryldimethicon-Kreuzpolymer,
- PEG-15/Laurylpolydimethylsiloxyethyldimethicon-Kreuzpolymer,
- Polyglycerin-3-polydimethylsiloxyethyldimethicon,
- Laurylpolyglycerin-3-polydimethylsiloxyethyldimethicon,
- Bisbutyldimethiconpolyglycerin-3,
- Dimethicon/Polyglycerin-3-Kreuzpolymer,
- Lauryldimethicon/Polyglycerin-3-Kreuzpolymer und
- Polyglycerin-3/Laurylpolydimethylsiloxyethyldimethicon-Kreuzpolymer.

2. Ölbasiertes Verdickungsmittel nach Anspruch 1, wobei die Silicon-modifizierte Polysaccharidverbindung ein Silicon-modifiziertes Pullulan der nachstehenden allgemeinen Formel (5) ist: 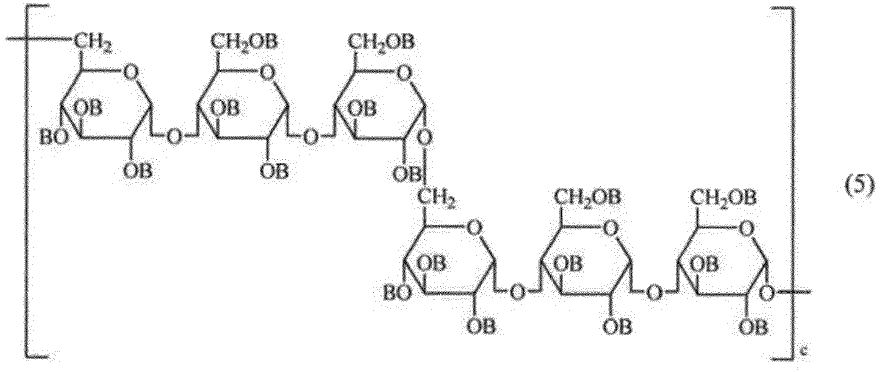 worin B ein Wasserstoffatom oder -CONH(CH₂)₃Si[OSi(CH₃)₃]₃ ist, der Substitutionsgrad 0,5 bis 2,5 beträgt und "c" eine Zahl von 100 bis 20.000 ist.

3. Ölbasiertes Verdickungsmittel nach Anspruch 1 oder 2, wobei das Verhältnis von Siliconemulgator zu Silicon-modifizierter Polysaccharidverbindung 0,5:99,5 bis 99,5:0,5 beträgt.

4. Ölbasiertes Verdickungsmittel nach Anspruch 3, wobei das Verhältnis von Siliconemulgator zu Silicon-modifizierter Polysaccharidverbindung 1:4 bis 4:1 beträgt.

5. Ölbasierte Verdickungszusammensetzung, die ein ölbasiertes Verdickungsmittel nach einem der Ansprüche 1 bis 4 und einen ölbasierten Inhaltsstoff umfasst.

6. Ölbasierte Verdickungszusammensetzung nach Anspruch 5, wobei der ölbasierte Inhaltsstoff eine flüssige Ölkomponente ist.

7. Ölbasierte Verdickungszusammensetzung nach Anspruch 5, wobei der ölbasierte Inhaltsstoff ein Siliconöl ist.

8. Ölbasierte Verdickungszusammensetzung nach Anspruch 6, wobei die ölbasierte flüssige Komponente aus niederviskosen Siliconölen mit einer kinetischen Viskosität von nicht mehr als 50 mm²/s, leichtem Isoparaffin und flüssigem leichtem Isoparaffin ausgewählt ist.

9. Ölbasierte Verdickungszusammensetzung nach einem der Ansprüche 5 bis 8, die aus einer Silicon-modifizierten Polysaccharidverbindung, einem Siliconemulgator und einem ölbasierten Inhaltsstoff besteht.

10. Ölbasierte Verdickungszusammensetzung nach einem der Ansprüche 5 bis 9, die 5 bis 30 Gew.-% eines ölbasierten Verdickungsmittels umfasst, das aus einer Silicon-modifizierten Polysaccharidverbindung und einem Siliconemulgator besteht.

11. Kosmetikpräparat, das mit einem ölbasierten Verdickungsmittel nach einem der Ansprüche 1 bis 4 und/oder einer ölbasierten Verdickungszusammensetzung nach einem der Ansprüche 5 bis 10 formuliert ist.

12. Verwendung eines ölbasierten Verdickungsmittels nach einem der Ansprüche 1 bis 4 und/oder einer ölbasierten Verdickungszusammensetzung nach einem der Ansprüche 5 bis 10 zum Formulieren eines Kosmetikpräparats.

## Revendications

1. Agent épaississant à base d'huile comprenant
(a) un composé polysaccharide modifié par de la silicone de formule générale (1) : 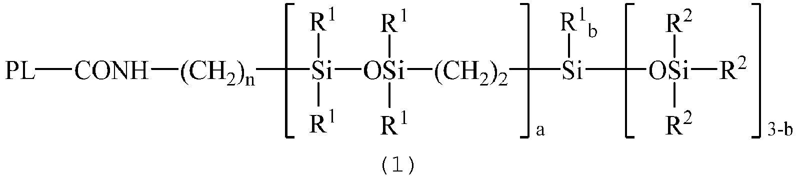 dans laquelle PL est un résidu glucose de pullulane ; les R¹ sont identiques ou différents et sont des groupes hydrocarbonés monovalents substitués ou non substitués de 1 à 10 atomes de carbone ; les R² sont identiques ou différents et sont des groupes hydrocarbonés monovalents substitués ou non substitués de 1 à 10 atomes de carbone ou un groupe siloxy de formule -OSi(R³)₃ ; les R³ sont identiques ou différents et sont des groupes hydrocarbonés monovalents substitués ou non substitués de 1 à 8 atomes de carbone ; "n" est un entier de 1 à 10, "a" vaut 0 ou 1, et "b" vaut 0, 1 ou 2 ; et le nombre moyen de liaisons ou le degré moyen de substitution du composé siliconé par motif sucre constitutif sur le composé polysaccharide est de 0,5 à 2,5, et lequel composé polysaccharide modifié par de la silicone a une masse moléculaire moyenne de 50 000 à 10 000 000, et
(b) un ou plusieurs agents émulsionnants siliconés choisis parmi
- un copolymère réticulé de diméthicone/PEG-10/15,
- un copolymère réticulé de PEG-15/lauryl-diméthicone,
- un copolymère réticulé de PEG-10/lauryl-diméthicone,
- un copolymère réticulé de PEG-15/lauryl-polydiméthylsiloxyéthyl-diméthicone,
- la polyglycéryl-3-polydiméthylsiloxyéthyl-diméthicone,
- la lauryl-polyglycéryl-3-polydiméthylsiloxyéthyl-diméthicone,
- le bis-butyl-diméthicone-polyglycéryle-3,
- un copolymère réticulé de diméthicone/polyglycérine-3,
- un copolymère réticulé de lauryl-diméthicone/ polyglycérine-3, et
- un copolymère réticulé de polyglycéryle-3/lauryl-polydiméthylsiloxyéthyl-diméthicone.

2. Agent épaississant à base d'huile selon la revendication 1, dans lequel le composé polysaccharide modifié par de la silicone est un pullulane modifié par de la silicone de formule générale (5) ci-dessous : 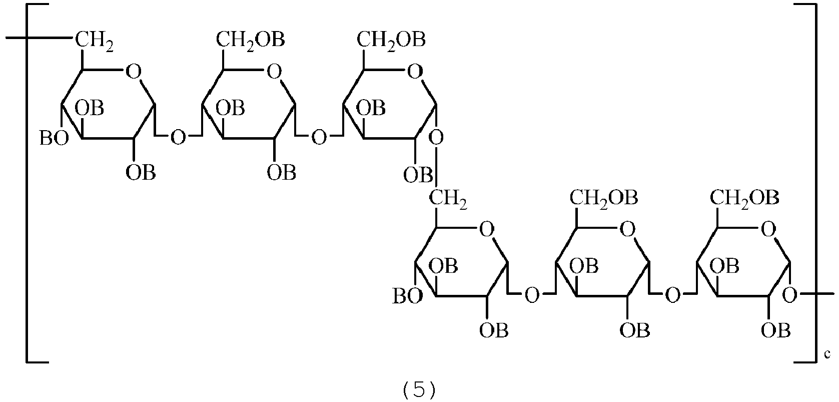 dans laquelle B est un atome d'hydrogène ou -CONH (CH₂) ₃Si[OSi(CH₃) ₃]₃, le degré de substitution est de 0,5 à 2,5, et "c" est un nombre de 100 à 20 000.

3. Agent épaississant à base d'huile selon la revendication 1 ou 2, dans lequel le rapport de l'agent émulsionnant siliconé au composé polysaccharide modifié par de la silicone est de 0,5/99,5 à 99,5/0,5.

4. Agent épaississant à base d'huile selon la revendication 3, dans lequel le rapport de l'agent émulsionnant siliconé au composé polysaccharide modifié par de la silicone est de 1/4 à 4/1.

5. Composition épaississante à base d'huile comprenant un agent épaississant à base de l'huile de l'une quelconque des revendications 1 à 4 et un ingrédient à base d'huile.

6. Composition épaississante à base d'huile selon la revendication 5, dans laquelle l'ingrédient à base d'huile est un composant huileux liquide.

7. Composition épaississante à base d'huile selon la revendication 5, dans laquelle l'ingrédient à base d'huile est une huile siliconée.

8. Composition épaississante à base d'huile selon la revendication 6, dans laquelle le composant liquide à base d'huile est choisi parmi les huiles siliconées faiblement visqueuses ayant une viscosité cinétique non supérieure à 50 mm²/s, l'isoparaffine légère et l'isoparaffine liquide légère.

9. Composition épaississante à base d'huile selon l'une quelconque des revendications 5 à 8, consistant en ledit composé polysaccharide modifié par de la silicone, l'agent émulsionnant siliconé et l'ingrédient à base d'huile.

10. Composition épaississante à base d'huile selon l'une quelconque des revendications 5 à 9, comprenant de 5 à 30 % en poids de l'agent épaississant à base d'huile consistant en ledit composé polysaccharide modifié par de la silicone et l'agent émulsionnant siliconé.

11. Préparation cosmétique formulée avec un agent épaississant à base d'huile selon l'une quelconque des revendications 1 à 4 et/ou une composition épaississante à base d'huile selon l'une quelconque des revendications 5 à 10.

12. Utilisation d'un agent épaississant à base d'huile selon l'une quelconque des revendications 1 à 4, et/ou d'une composition épaississante à base d'huile selon l'une quelconque des revendications 5 à 10, pour la formulation d'une préparation cosmétique.
